# EUROPÄISCHE PATENTANMELDUNG

(11) **EP 0 769 489 A1**
(43) Veröffentlichungstag der Anmeldung: **23.04.1997**
(21) Anmeldenummer: 96116044.7
(22) Anmeldetag: 07.10.1996
(51) Int. Cl.: C07C 209/68

(54) **Verfahren zur Herstellung von (Bi)Cycloalkylanilinen**

(30) Priorität: 20.10.1995 DE 19539113
(71) Anmelder: BAYER AG, 51368 Leverkusen (DE)
(72) Erfinder: Bielefeldt, Dietmar, Dr., 40883 Ratingen (DE); Elbe, Hans-Ludwig, Dr., 42329 Wuppertal (DE); Puppe, Lothar, Dr., 51399 Burscheid (DE)

(57) **Zusammenfassung**

Nach einem neuen Verfahren lassen sich in 2-Position durch (Bi)Cycloalkyl substituierte Aniline der Formel 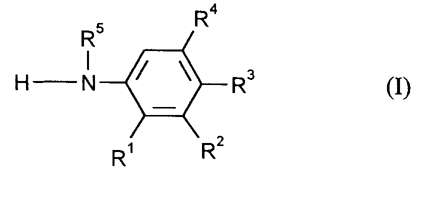 in welcher
R¹, R², R³, R⁴ und R⁵ die in der Beschreibung angegebenen Bedeutungen haben,
herstellen, indem man Aniline der Formel 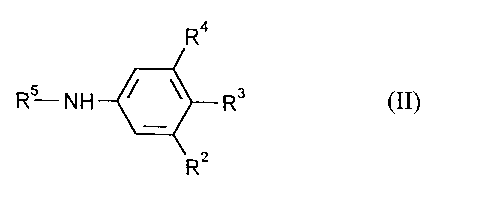 in welcher
R², R³, R⁴ und R⁵ die in der Beschreibung angegebenen Bedeutungen haben,
mit (Bi)Cycloalkenen der Formel

H-R⁶ (III)

in welcher
R⁶ die in der Beschreibung angegebene Bedeutung hat,
in Gegenwart eines mit Seltenerd-Kationen dotierten Zeolith-Katalysators gegebenenfalls in Gegenwart eines Verdünnungsmittels bei Temperaturen zwischen 100°C und 500°C umsetzt.

## Beschreibung

Die vorliegende Erfindung betrifft ein neues Verfahren zur Herstellung von in 2-Position durch (Bi)Cycloalkyl substituierten Anilinen, die als Zwischenprodukte zur Synthese von Wirkstoffen mit fungiziden Eigenschaften verwendet werden können.

Es ist bereits bekannt, daß sich zahlreiche Olefine in Gegenwart von Aluminiumkatalysatoren an aromatische Amine addieren lassen (vergl. Houben-Weyl "Methoden der Organischen Chemie", Band XI/1, Seite 1021 ff. und US-A 2 814 646). So gelingt es zum Beispiel mit Hilfe von Aluminium-anilid als Katalysator, Aniline in 2- und 6-Stellung zu alkylieren. Nachteilig an diesem Verfahren ist aber, daß selbst bei vorzeitigem Abbrechen der Umsetzung jeweils Gemische aus 2-Alkyl-anilin und 2,6-Dialkyl-anilin entstehen. Eine Trennung des Komponenten ist aufwendig.

Weiterhin ist schon bekannt, daß Aniline in Gegenwart von üblichen Friedel-Crafts-Katalysatoren, wie Aluminiumchlorid, am Aromaten alkyliert werden können (vergl. US-A 3 275 690). Auch hierbei werden aber stets Gemische aus isomeren mono-substituierten Anilinen und di-substituierten Anilinen erhalten, aus denen die nur in 2-Stellung alkylierten Produkte schwierig in reiner Form isolierbar sind. Ungünstig ist auch, daß die verwendeten Katalysatoren hydrolyseempfindlich sind, und zwar insbesondere dann, wenn noch Co-Katalysatoren, wie Natrium, hinzugefügt wurden.

Wie ferner aus der Literatur hervorgeht, gelingt eine direkte Alkylierung von Anilinen in Gegenwart von Friedel-Crafts-Katalysatoren, wenn es sich bei der Olefin-Komponente um Ethen handelt. Werden hingegen verzweigte oder cyclische Olefine verwendet, sinkt die Reaktionsrate mit dem Substitutionsgrad des Olefins dramatisch (vergl. Applied Cat. 63, 117 (1990)).

Schließlich ist auch bekannt, daß eine überwiegende mono-Alkylierung von Anilinen möglich ist, wenn H-Y-Zeolithe oder Gemische aus 13 % Aluminiumoxid und 87 % Siliziumdioxid als Katalysatoren fungieren. Allerdings fällt auch hierbei ein Gemisch aus meta-, ortho- und para-substituierten Alkylierungsprodukten an. Die reinen Isomeren sind wiederum nur durch umständliche Trennung und in relativ geringen Ausbeuten zugänglich.

Es wurde nun gefunden, daß man in 2-Position durch (Bi)Cycloalkyl substituierte Aniline der Formel 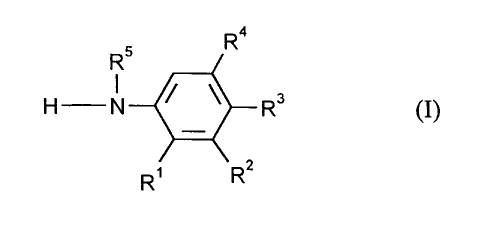 in welcher
- R¹: für Cycloalkyl mit 5 bis 12 Kohlenstoffatomen steht, wobei jeder dieser Reste einfach bis dreifach, gleichartig oder verschieden durch Alkyl mit 1 bis 4 Kohlenstoffatomen substituiert sein kann, oder
- R¹: für Bicycloalkyl mit 7 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
- R², R³ und R⁴: unabhängig voneinander für Wasserstoff, geradkettiges oder verzweigtes Alkyl mit 1 bis 6 Kohlenstoffatomen, Halogen, Cyano, Halogenalkyl mit 1 bis 4 Kohlenstoffatomen und 1 bis 5 gleichen oder verschiedenen Halogenatomen, Alkoxy mit 1 bis 6 Kohlenstoffatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen oder für Alkoxycarbonyl mit 1 bis 6 Kohlenstoffatomen in der Alkoxygruppe stehen, und
- R⁵: für Wasserstoff, Alkylcarbonyl mit 1 bis 4 Kohlenstoffatomen in der Alkylgruppe oder für Halogenalkylcarbonyl mit 1 oder 2 Kohlenstoffatomen und 1 bis 5 gleichen oder verschiedenen Halogenatomen in der Halogenalkylgruppe steht,
erhält, indem man Aniline der Formel 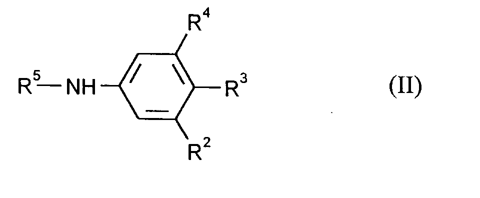 in welcher
- R², R³, R⁴ und R⁵: die oben angegebenen Bedeutungen haben,
mit (Bi)Cycloalkenen der Formel

H-R⁶ (III)

in welcher
- R⁶: für Cycloalkenyl mit 5 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
oder
- R⁶: für Bicycloalkenyl mit 7 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
in Gegenwart eines mit Seltenerd-Kationen dotierten Zeolith-Katalysators gegebenenfalls in Gegenwart eines Verdünnungsmittels bei Temperaturen zwischen 100°C und 500°C umsetzt.

Es ist als äußerst überraschend zu bezeichnen, daß sich nach dem erfindungsgemäßen Verfahren in ortho-Stellung durch Cycloalkyl oder Bicycloalkyl substituierte Aniline mit hoher Selektivität herstellen lassen. Aufgrund des bekannten Standes der Technik war nämlich damit zu rechnen, daß auch bei dieser Synthesemethode jeweils Gemische aus mono-substituierten Isomeren und mehrfach cycloalkylierten Produkten anfallen würden.

Das erfindungsgemäße Verfahren weist eine Reihe von Vorteilen auf. So ermöglicht es die Herstellung einer Vielzahl von in 2-Stellung durch Cycloalkyl oder Bicycloalkyl substituierten Anilinen mit hoher Selektivität und sehr guter Ausbeute. Günstig ist auch, daß sowohl die Ausgangsstoffe als auch die Katalysatoren in einfacher Weise zugänglich sind und auch in größeren Mengen zur Verfügung stehen. Von besonderem Vorteil ist schließlich, daß die eingesetzten Katalysatoren reaktiviert werden können und wiederholt einsetzbar sind.

Verwendet man Anilin und Bicyclo[2,2,1]-hept-2-en als Ausgangsstoffe und einen mit Seltenerdchlorid dotierten Zeolith-Katalysator, so kann der Verlauf des erfindungsgemäßen Verfahrens durch das folgende Formelschema veranschaulicht werden.

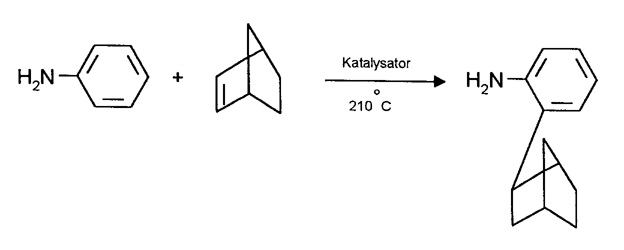

Die bei der Durchführung des erfindungsgemäßen Verfahrens als Ausgangsstoffe verwendeten Aniline sind durch die Formel (II) allgemein definiert. In dieser Formel stehen
- R², R³ und R⁴: unabhängig voneinander vorzugsweise für Wasserstoff, Fluor, Chlor, Brom, Cyano, geradkettiges oder verzweigtes Alkyl mit 1 bis 4 Kohlenstoffatomen, für Alkoxy mit 1 bis 4 Kohlenstoffatomen oder für Alkoxycarbonyl mit 1 bis 4 Kohlenstoffatomen in der Alkoxygruppe, und
- R⁵: steht vorzugsweise für Wasserstoff, Alkylcarbonyl mit 1 oder 2 Kohlenstoffatomen in der Alkylgruppe oder für Halogenalkylcarbonyl mit 1 oder 2 Kohlenstoffatomen und 1 bis 3 Fluor-, Chlor- und/oder Bromatomen in der Halogenalkylgruppe.

Besonders bevorzugt sind Aniline der Formel (II), in denen
- R², R³ und R⁴: unabhängig voneinander für Wasserstoff, Fluor, Chlor, Brom, Cyano, Methyl, Ethyl, n-Propyl Isopropyl, n-Butyl, i-Butyl, sek.-Butyl, tert.-Butyl, Methoxy, Ethoxy, Methoxycarbonyl oder Ethoxycarbonyl stehen, und
- R⁵: für Wasserstoff, Methylcarbonyl, Ethylcarbonyl oder für Trifluormethylcarbonyl steht.

Die Aniline der Formel (II) sind bekannt oder lassen sich nach bekannten Methoden herstellen.

Die bei der Durchführung des erfindungsgemäßen Verfahrens als Reaktionskomponenten benötigten (Bi)Cycloalkene sind durch die Formel (III) allgemein definiert.

In dieser Formel steht
- R⁶: vorzugsweise für Cycloalkenyl mit 5 bis 12 Kohlenstoffatomen, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl, oder
- R⁶: steht vorzugsweise für Bicycloalkenyl mit 7 bis 12 Kohlenstoffatomen, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl.

Besonders bevorzugt sind (Bi)Cycloalkene der Formel (III), in denen
- R⁶: für Cyclopentenyl, Cyclohexenyl, Cycloheptenyl, Cyclooctenyl, Cyclododecenyl, Bicyclo[2,2,1]-hept-2-enyl oder Bicyclo[2,2,2]oct-2-enyl steht, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl.

Die (Bi)Cycloalkene der Formel (III) sind bekannte Verbindungen der organischen Chemie.

Als Katalysatoren kommen bei der Durchführung des erfindungsgemäßen Verfahrens alle weitporigen Zeolithe in Frage, die saure Zentren enthalten und mit Seltenerd-Kationen dotiert sind. Vorzugsweise verwendbar sind z.B. derartige Zeolithe der Strukturtypen Faujasit, Mordenit, Zeolith L, Zeolith β, Zeolith Ω und EMT. Bevorzugt zur Dotierung verwendbare Seltene Erdsalze sind Gemische, die einen hohen Anteil an Lanthan- und/oder Cersalzen enthalten. Besonders gut geeignet sind Zeolithe der genannten Typen, die Protonen aufweisen und die saure Zentren aus einem Ionenaustausch mit Lanthan- oder Cer-reicher Seltener Erden und/oder mit Ammonium-Salzen enthalten und nach der Dotierung einer thermischen Behandlung unterworfen wurden.

Die bei der Durchführung des erfindungsgemäßen Verfahrens als Katalysatoren benötigten Zeolithe sind bekannt oder lassen sich nach bekannten Methoden herstellen. So wird die Herstellung von Zeolithen ausführlich beschrieben in der Monographie von D. W. Breck "Zeolite Molecular Sieves, Structure, Chemistry and Use", J. Wiley & Sons, New York 1974. Weitere Angaben zur Herstellung von Zeolithen finden sich in der Monographie von P.A. Jacobs und J.A. Martens "Synthesis of High Silica Aluminosilicate Zeolites", Studies in Surface Science and Catalysis, Vol. 33, Ed. B. Delmon and J.T. Yates, Elsevier, Amsterdam-Oxford-New York-Tokyo, 1987.

Die sauren Zentren der Zeolithe werden bevorzugt dadurch hergestellt, daß man Metallionen gegen Ammoniumionen austauscht und den so behandelten Zeolith anschließend calciniert. Eine Wiederholung des Austauschverfahrens mit nachgeschalteter Calcinierung unter definierten Bedingungen führt bei Zeolithen des Faujasit-Typs unter Verminderung des Aluminiumgehaltes und der Anzahl der sauren Zentren zu sogenannten ultrastabilen Zeolithen, die durch den Dealuminierungsvorgang thermisch und hydrothermal stabiler werden.

Eine weitere Möglichkeit zur Erzeugung von sauren Zentren durch den Austausch von Protonen besteht darin, bei Zeolithen, die ein Siliziumdioxid/Aluminiumoxid-Verhältnis von mindestens 5 aufweisen, den Austauschvorgang mit Mineralsäuren vorzunehmen.

Stark saure Zentren lassen sich vor allem bei Zeolithen vom Faujasit-Typ auch dadurch erzeugen, daß man den Ionenaustausch mit Salzen von einzelnen Seltenen Erden oder von Seltenerd-Gemischen vornimmt. Durch Variation des Ionenaustauschgrades kann die Acidität des Katalysators verändert werden. Besonders hohe Austauschgrade lassen sich dadurch erzielen, daß zwischen den einzelnen Austausch-Schritten eine Zwischencalcinierung bei mindestens 350°C erfolgt. Nach dieser Zwischencalcinierung wird ein erneuter Ionenaustausch und die abschließende Calcinierung vorgenommen.

Als Verdünnungsmittel kommen bei der Durchführung des erfindungsgemäßen Verfahrens alle inerten organischen Solventien in Betracht. Vorzugsweise verwendbar sind aliphatische und cycloaliphatische Kohlenwasserstoffe, wie Hexan, Heptan, Cyclohexan und Methylcyclohexan. Im allgemeinen erübrigt sich jedoch die Verwendung eines Verdünnungsmittels.

Die Reaktionstemperaturen können bei der Durchführung des erfindungsgemäßen Verfahrens in einem größeren Bereich variiert werden. Im allgemeinen arbeitet man bei Temperaturen zwischen 100°C und 500°C, vorzugsweise zwischen 180°C und 350°C. Bei der Durchführung des erfindungsgemäßen Verfahrens arbeitet man unter Atmosphärendruck oder vermindertem oder unter erhöhtem Druck. Im allgemeinen arbeitet man unter Drucken zwischen 0,01 bar und 300 bar, vorzugsweise zwischen 1 bar und 30 bar.

Bei der Durchführung des erfindungsgemäßen Verfahrens kann man unter Inertgasatmosphäre arbeiten, vorzugsweise unter Stickstoff oder Argon.

Bei der Durchführung des erfindungsgemäßen Verfahrens setzt man auf 1 mol an Anilin der Formel (II) im allgemeinen 1 bis 2 mol an (Bi)Cycloalken der Formel (III) sowie eine praktisch beliebige Menge an Katalysator ein. Es ist jedoch auch möglich, die Reaktionskomponenten in anderen molaren Mengen einzusetzen.

Das erfindungsgemäße Verfahren kann sowohl kontinuierlich als auch diskontinuierlich durchgeführt werden. Bei der diskontinuierlichen Arbeitsweise geht man im allgemeinen so vor, daß man die Reaktionskomponenten und den Katalysator in ein Druckgefäß gibt, dann das Gefäß verschließt, auf die gewünschte Temperatur erhitzt und unter dem sich einstellenden Eigendruck reagieren läßt. Die Aufarbeitung erfolgt nach üblichen Methoden. Im allgemeinen geht man so vor, daß man das Reaktionsgemisch filtriert und destilliert. Bei der kontinuierlichen Arbeitsweise geht man im allgemeinen so vor, daß man einen senkrecht stehenden Quarzreaktor mit einer Mischung aus Quarzbruch und Katalysator füllt, die Apparatur unter Durchleiten eines Stickstoffstromes von unten nach oben spült und den Katalysator bei erhöhter Temperatur trocknet und dann die Reaktionskomponenten von oben kontinuierlich zuführt. Das aus dem Reaktor austretende Gemisch wird kondensiert und gegebenenfalls nach vorheriger Filtration aufgearbeitet, also zum Beispiel destilliert.

Nach Beendigung der erfindungsgemäßen Umsetzung kann der Katalysator wieder reaktiviert werden, indem man den Katalysator mit einem organischen Lösungsmittel, wie zum Beispiel Essigsäureethylester, wäscht, in einen senkrecht stehenden Quarzreaktor gibt und unter Durchleiten eines Luftstromes mehrere Stunden auf Temperaturen zwischen 400°C und 550°C erhitzt. Der so behandelte Katalysator kann erneut für erfindungsgemäße Umsetzungen verwendet werden.

Die nach dem erfindungsgemäßen Verfahren herstellbaren, in 2-Position durch Cycloalkyl oder Bicycloalkyl substituierten Aniline der Formel (I) sind wertvolle Zwischenprodukte zur Herstellung von Wirkstoffen mit fungiziden Eigenschaften (vergl. US-A 5 223 526 und EP-A 0 545 099). So erhält man zum Beispiel die fungizid wirksame Verbindung der Formel 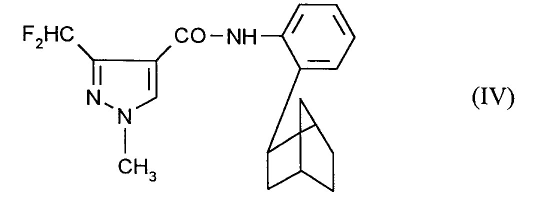 indem man das 2-(Bicyclo[2,2,1]-hept-2-yl)-anilin der Formel 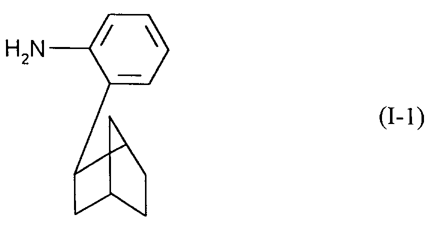 mit 1-Methyl-3-difluormethyl-pyrazol-4-yl-carbonsäurechlorid der Formel 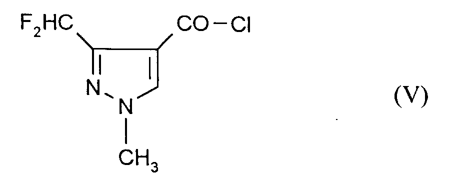 in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

### Beispiele

### Herstellung eines Katalysators:

### Beispiel A

In einem 3-Liter-Behälter mit Rührwerk und Heizung werden 2,5 Liter Wasser und 500 g Zeolith Y-Pulver (Siliziumdioxid/Aluminiumoxid = 4,8, bezogen auf wasserfreies Material) vorgelegt. Danach werden 235 ml einer wäßrigen Seltenerdchlorid-Lösung (lanthanreich, mit einem Gehalt von 523 g Seltenerdchlorid pro Liter Lösung; Fa. Treibacher) zugegeben. Das Gemisch wird auf 80°C aufgeheizt und 6 Stunden bei dieser Temperatur gerührt. Anschließend filtriert man den Feststoff ab, wäscht mit Wasser nach und calciniert 6 Stunden bei 500°C (erster Austausch und Zwischencalcinierung). Der erhaltene Feststoff wird mit 2 Litern Wasser und mit 250 ml der oben erwähnten, wäßrigen Seltenerdchlorid-Lösung versetzt. Das Gemisch wird auf 80°C aufgeheizt und 6 Stunden bei dieser Temperatur gerührt. Danach wird der Feststoff abfiltriert, mit Wasser gewaschen und bei 100°C getrocknet. Anschließend calciniert man das Material 12 Stunden bei 500°C.

### Beispiel 1

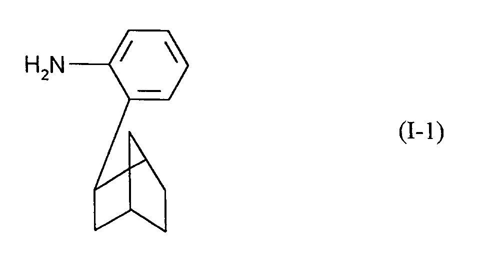

In einen Autoklaven mit einem Fassungsvermögen von 100 ml werden bei Raumtemperatur 28,2 g (0,3 mol) Bicyclo[2,2,1]-hept-2-en, 27,9 g (0,3 mol) Anilin und 9,2 g des im Beispiel A beschriebenen Zeolith-Katalysators gegeben. Danach wird das Gefäß verschlossen und 10 Stunden auf 210°C erhitzt, wobei die Komponenten unter dem sich einstellenden Eigendruck reagieren. Danach läßt man auf Raumtemperatur abkühlen, filtriert das Reaktionsgemisch und analysiert das Filtrat gaschromatographisch. Danach ergibt sich, daß der Umsatz zu 96,1 % stattgefunden hat. Das 2-(Bicyclo[2,2,1]-hept-2-yl)-anilin ist mit einer Selektivität von 83,1 % entstanden.

Nach der im Beispiel 1 aufgeführten Methode werden auch die in der folgenden Tabelle aufgeführten Verbindungen der Formel 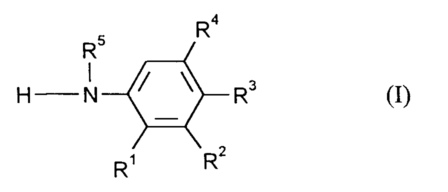 hergestellt.

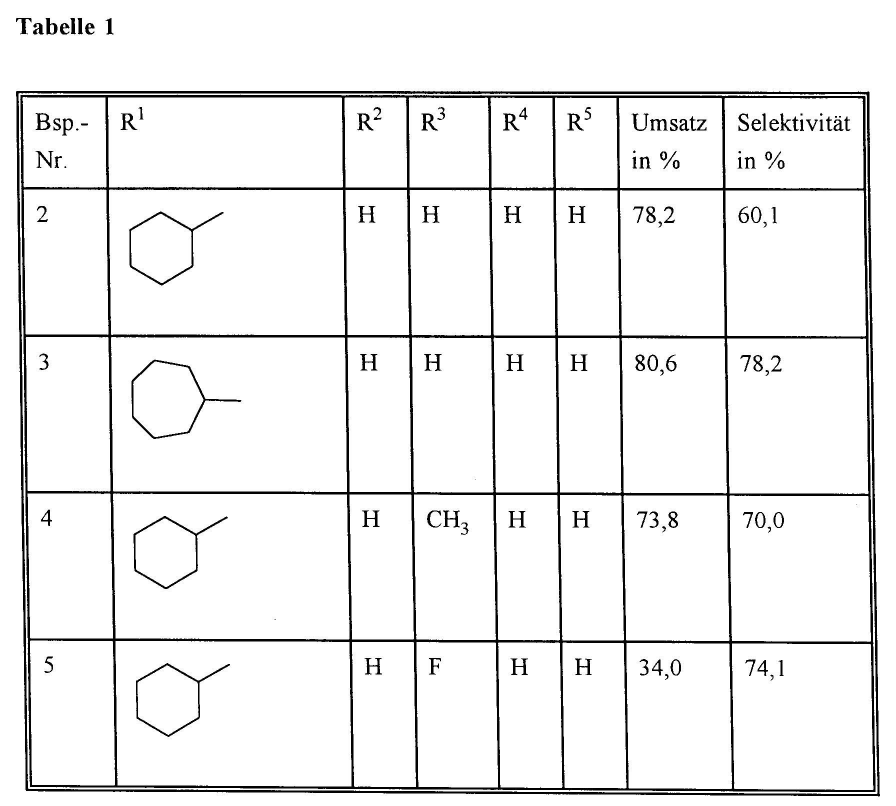

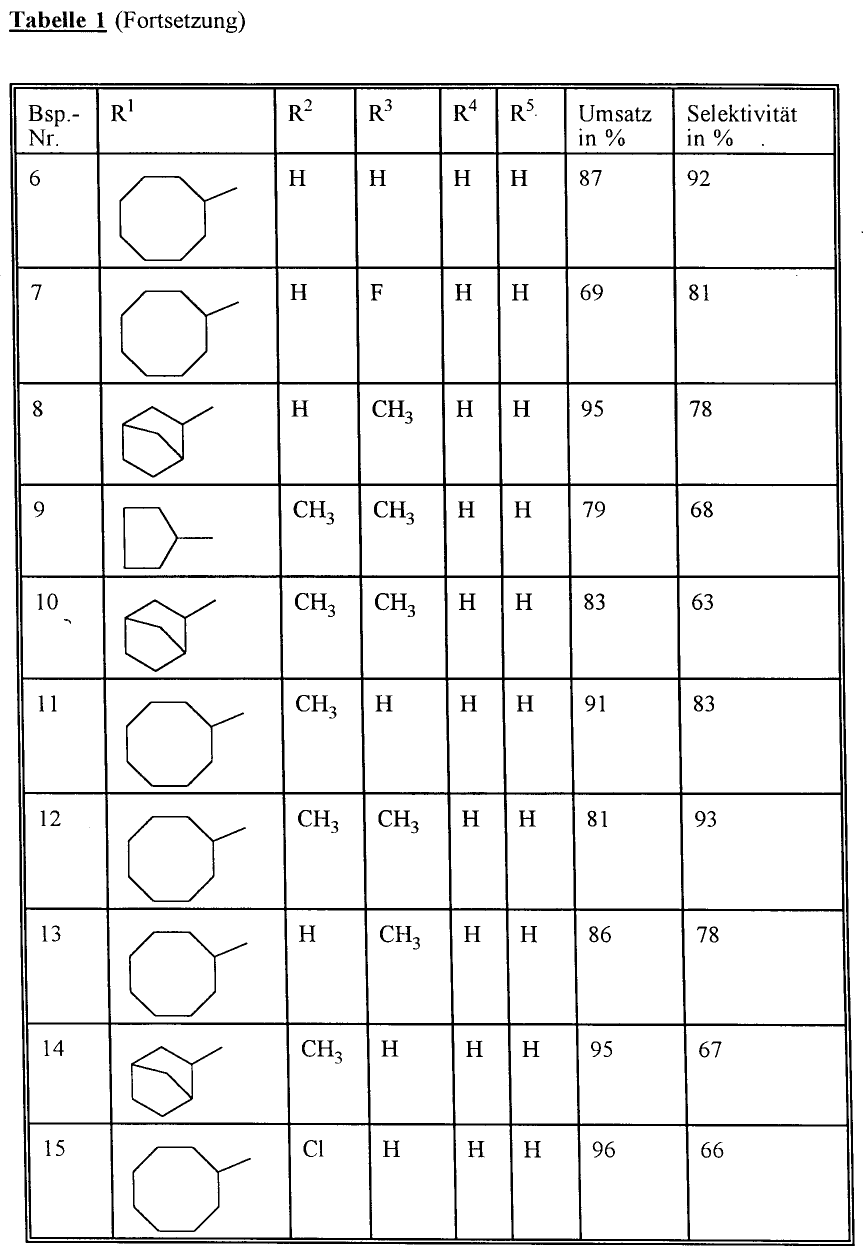

### Beispiel 16

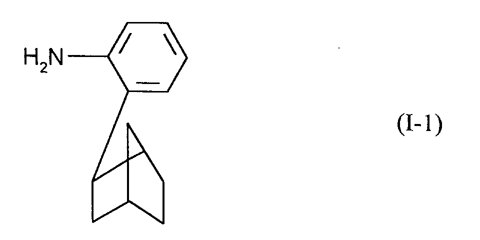

### Kontinuierliche Arbeitsweise:

Ein senkrecht stehender, beheizbarer Quarzreaktor wird mit einer Mischung aus 100 ml des im Beispiel A beschriebenen Zeolith-Katalysators und 100 ml Quarzbruch gefüllt. Danach heizt man die Apparatur auf 350°C auf und leitet zum Spülen und Trocknen des Katalysators einen Stickstoffstrom von unten nach oben hindurch. Anschließend wird bei 300°C ein Gemisch aus gleichen molaren Mengen an Anilin und Bicyclo[2,2,1]-hept-2-en mit Hilfe einer Dosierpumpe kontinuierlich von oben nach unten durch den Reaktor geleitet, und zwar mit einer solchen Geschwindigkeit, daß die Katalysatorbelastung bei etwa 800 g/l und pro Stunde liegt. Das aus dem Reaktor austretende Gemisch wird bei Temperaturen zwischen 0°C und 20°C kondensiert und gaschromatographisch untersucht. Daraus ergibt sich, daß der Umsatz zu 100 % stattgefunden hat. Das 2-(Bicyclo[2,2,1]-hept-2-yl)-anilin ist mit einer Selektivität von 69 % entstanden.

### Reaktivierung des Katalysators:

Nach dem Ende der im Beispiel 1 beschriebenen Umsetzung wird der feste Katalysator abfiltriert, mit Essigsäureethylester gewaschen und anschließend in einem senkrecht stehenden Quarzreaktor 16 Stunden lang auf 500°C erhitzt; dabei wird kontinuierlich ein Luftstrom von 60 dm/h durch die Apparatur geleitet. Die Aktivität des so reaktivierten Katalysators ist unwesentlich geringer als diejenige eines frisch eingesetzten.

## Patentansprüche

1. Verfahren zur Herstellung von in 2-Position durch (Bi)Cycloalkyl substituierten Anilinen der Formel 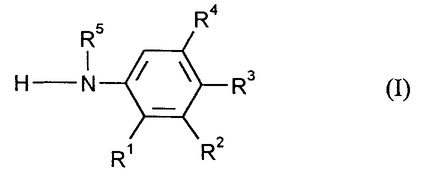 in welcher
R¹ für Cycloalkyl mit 5 bis 12 Kohlenstoffatomen steht, wobei jeder dieser Reste einfach bis dreifach, gleichartig oder verschieden durch Alkyl mit 1 bis 4 Kohlenstoffatomen substituiert sein kann, oder
R¹ für Bicycloalkyl mit 7 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
R², R³ und R⁴ unabhängig voneinander für Wasserstoff, geradkettiges oder verzweigtes Alkyl mit 1 bis 6 Kohlenstoffatomen, Halogen, Cyano, Halogenalkyl mit 1 bis 4 Kohlenstoffatomen und 1 bis 5 gleichen oder verschiedenen Halogenatomen, Alkoxy mit 1 bis 6 Kohlenstoffatomen, Halogenalkoxy mit 1 bis 4 Kohlenstoffatomen und 1 bis 5 gleichen oder verschiedenen Halogenatomen, Halogenalkylthio mit 1 bis 4 Kohlenstoffatomen und 1 bis 5 gleichen oder verschiedenen Halogenatomen oder für Alkoxycarbonyl mit 1 bis 6 Kohlenstoffatomen in der Alkoxygruppe stehen, und
R⁵ für Wasserstoff, Alkylcarbonyl mit 1 bis 4 Kohlenstoffatomen in der Alkylgruppe oder für Halogenalkylcarbonyl mit 1 oder 2 Kohlenstoffatomen und 1 bis 5 gleichen oder verschiedenen Halogenatomen in der Halogenalkylgruppe steht,
dadurch gekennzeichnet, daß man Aniline der Formel 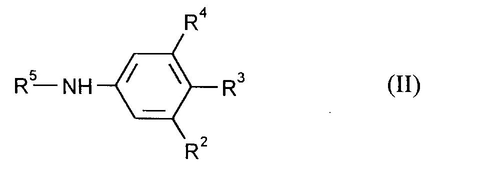 in welcher
R², R³, R⁴ und R⁵ die oben angegebenen Bedeutungen haben,
mit (Bi)Cycloalkenen der Formel
H-R⁶ (III)
in welcher
R⁶ für Cycloalkenyl mit 5 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
oder
R⁶ für Bicycloalkenyl mit 7 bis 12 Kohlenstoffatomen steht, wobei jeder dieser Reste einfach bis dreifach, gleichartig oder verschieden durch Alkyl mit 1 bis 4 Kohlenstoffatomen substituiert sein kann,
in Gegenwart eines mit Seltenerd-Kationen dotierten Zeolith-Katalysators gegebenenfalls in Gegenwart eines Verdünnungsmittels bei Temperaturen zwischen 100°C und 500°C umsetzt.

2. Verfahren gemäß Anspruch 1, dadurch gekennzeichnet, daß man Aniline der Formel (II) einsetzt, in denen
R², R³ und R⁴ unabhängig voneinander für Wasserstoff, Fluor, Chlor, Brom, Cyano, geradkettiges oder verzweigtes Alkyl mit 1 bis 4 Kohlenstoffatomen, für Alkoxy mit 1 bis 4 Kohlenstoffatomen oder für Alkoxycarbonyl mit 1 bis 4 Kohlenstoffatomen in der Alkoxygruppe stehen und
R⁵ für Wasserstoff, Alkylcarbonyl mit 1 oder 2 Kohlenstoffatomen in der Alkylgruppe oder für Halogenalkylcarbonyl mit 1 oder 2 Kohlenstoffatomen und 1 bis 3 Fluor-, Chlor- und/oder Bromatomen in der Halogenalkylgruppe steht.

3. Verfahren gemäß Anspruch 1, dadurch gekennzeichnet, daß man Aniline der Formel (II) einsetzt, in denen
R², R³ und R⁴ unabhängig voneinander für Wasserstoff, Fluor, Chlor, Brom, Cyano, Methyl, Ethyl, n-Propyl Isopropyl, n-Butyl, i-Butyl, sek.-Butyl, tert.-Butyl, Methoxy, Ethoxy, Methoxycarbonyl oder Ethoxycarbonyl stehen, und
R⁵ für Wasserstoff, Methylcarbonyl, Ethylcarbonyl oder für Trifluormethylcarbonyl steht.

4. Verfahren gemäß Anspruch 1, dadurch gekennzeichnet, daß man (Bi)Cycloalkene der Formel (III) einsetzt, in denen
R⁶ für Cycloalkenyl mit 5 bis 12 Kohlenstoffatomen steht, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl, oder
R⁶ für Bicycloalkenyl mit 7 bis 12 Kohlenstoffatomen steht, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl.

5. Verfahren gemäß Anspruch 1, dadurch gekennzeichnet, daß man (Bi)Cycloalkene der Formel (III) einsetzt, in denen
R⁶ für Cyclopentenyl, Cyclohexenyl, Cycloheptenyl, Cyclooctenyl, Cyclododecenyl, Bicyclo[2,2,1]-hept-2-enyl oder Bicyclo[2,2,2]oct-2-enyl steht, wobei jeder dieser Reste einfach bis dreifach, gleichartig oder verschieden substituiert sein kann durch Methyl, Ethyl, n-Propyl, Isopropyl, n-Butyl, i-Butyl, sek.-Butyl und/oder tert.-Butyl.

6. Verfahren gemäß Anspruch 1, dadurch gekennzeichnet, daß man bei Temperaturen zwischen 180°C und 350°C arbeitet.

7. Verfahren gemäß Anspruch 1, dadurch gekennzeichnet, daß man unter Drucken zwischen 0,01 bar und 300 bar arbeitet.
